(19) Europäisches Patentamt
European Patent Office
Office européen des brevets

(11) **EP 3 199 973 A1**

(12) **EUROPEAN PATENT APPLICATION**

(43) Date of publication:
**02.08.2017 Bulletin 2017/31**

(51) Int Cl.:
**G01S 7/52** *(2006.01)*     **A61B 5/00** *(2006.01)*
**A61B 8/13** *(2006.01)*     **G01N 29/24** *(2006.01)*

(21) Application number: **17155970.1**

(22) Date of filing: **14.02.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.02.2010   JP 2010031171**

(62) Document number(s) of the earlier application(s) in accordance with Art. 76 EPC:
**11706365.1 / 2 536 338**

(71) Applicant: **Canon Kabushiki Kaisha**
**Tokyo 146-8501 (JP)**

(72) Inventors:
• **NAGAE, Kenichi**
  **Tokyo 146-8501 (JP)**
• **BABA, Yoshitaka**
  **Tokyo 146-8501 (JP)**

(74) Representative: **TBK**
**Bavariaring 4-6**
**80336 München (DE)**

Remarks:
This application was filed on 14.02.2017 as a divisional application to the application mentioned under INID code 62.

(54) **SUBJECT INFORMATION PROCESSING APPARATUS USING ACOUSTIC WAVES RECEIVED FROM THE SUBJECT**

(57) There is provided a subject information processing apparatus including a first acoustic conversion element that receives an acoustic wave having a first frequency reflected inside a subject and converts the acoustic wave into an analog signal, a second acoustic conversion element that receives an acoustic wave having a second frequency generated when light is irradiated to a subject and converts the acoustic wave into an analog signal, an A/D converter that converts the analog signal into a digital signal, a data memory that retains the digital signal, and a controller that instructs the data memory to output the digital signal at a predetermined sampling frequency for each receiving axis.

FIG. 1

**Description**

[Technical Field]

The present invention relates to a subject information processing apparatus that acquires information inside a subject using an acoustic wave received from the subject.

[Background Art]

As a technique of acquiring image data by transmitting light or an acoustic wave (an ultrasonic wave) to the inside of a subject, receiving an acoustic wave emitted from the subject, and converting the acoustic wave into an electrical signal, there are an ultrasonic wave echo and a photoacoustic tomography. The ultrasonic wave echo refers to a technique of creating an image by transmitting an ultrasonic wave that is an acoustic wave to a subject and receiving a reflected wave. The photoacoustic tomography refers to a technique of creating an image by transmitting light energy to the inside of a subject and an acoustic wave (typically, an ultrasonic wave) generated when the subject absorbs light energy and so is thermally insulated and expanded. The acoustic wave generated at this time is also called a photoacoustic wave.

The ultrasonic wave echo is generally used for a biological object in a medical field. The ultrasonic wave echo can convert a difference of acoustic impedance inside a biological object into an image and is being spread as a diagnosis means that is very useful in safety, convenience, and real time property. Meanwhile, the photoacoustic tomography that transmits light energy and receives a photoacoustic wave also attracts attention due to safety or a possibility of delineating a difference of an optical absorption coefficient of an organ.

In both the ultrasonic wave echo and the photoacoustic wave tomography, a time difference corresponding to an attention position is conferred and added to each of signals converted into electrical signals (received signals) by a plurality of acoustic conversion elements. By further performing a process, such as filtering, on the added signal, each of the received signals can be converted into an image.

As described above, the ultrasonic wave echo and the photoacoustic tomography can convert a different characteristic inside the biological object into an image, respectively. On a reception process, both techniques receive and process the acoustic wave emitted from the biological object through a plurality of acoustic conversion elements. For this reason, an attempt to implement both techniques in a single system in real time has been made. For example, Patent Literature 1 discloses a system that measures the ultrasonic wave echo and the photoacoustic tomography in a common circuit. Further, in Non Patent Literature 1, a difference of an optical absorption coefficient of an organ is detected at the depth of about 15 mm by using an acoustic conversion element having a center frequency of 1 MHz.

[Citation List]

[Patent Literature]

[PTL 1] Japanese Patent Application Laid-Open No. 2005-21380

[Non Patent Literature]

[NPL 1] Srirang Manohar, Susanne E. Vaartjes, Johan C. G. van Hespen, Joost M. Klaase, Frank M. van den Engh, Wiendelt Steenbergen, and Ton G. van Leeuwen, "Initial results of in vivo non-invasive cancer imaging in the human breast using near-infrared photoacoustics", Opt. Express 15, 12277-12285 (2007)

[Summary of Invention]

[Technical Problem]

As described above, in the reception process of the ultrasonic wave echo and the photoacoustic tomography, a time difference corresponding to the attention position is conferred and added to a signal that has been converted into an electrical signal using a plurality of acoustic conversion elements. Through such a process, it is possible to selectively extract a signal of an acoustic wave that has arrived at the acoustic conversion element from the attention point.

At the time of transmitting the ultrasonic wave echo, by conferring a time difference corresponding to a target convergence point to an electrical signal (a transmission signal) to be output to each acoustic conversion element and transmitting the ultrasonic wave, an ultrasonic transmission beam converged to a target point or in a target direction can be formed. As described above, in the ultrasonic wave echo, in both reception and transmission, it is possible to transmit and receive the ultrasonic wave converged to a target point or in a target direction. For this reason, by making an attention point or direction of transmission correspond to an attention point or direction of reception, the transmitted ultrasonic wave energy can be utilized in efficiently imaging the inside of the subject.

Meanwhile, in the photoacoustic tomography, transmission of light energy is performed by irradiating light to the subject. However, if the subject is a strong scatterer such as a biological object, it is very difficult to converge light in an arbitrary position inside the subject. For this reason, in the photoacoustic tomography, the light energy transmitted cannot be converged as in transmission of the ultrasonic wave echo, and the light energy that is scattered and spread from the incident position becomes a transmission range.

In the case of a technique of performing the reception process by making a transmission direction correspond to a reception direction as in the conventional ultrasonic wave echo in a situation in which the light energy that is scattered and spread inside the subject becomes the transmission range as described above, an area drawing attention at the time of the reception process becomes narrower than a range in which the transmitted light energy is spread. For this reason, the transmitted light energy cannot be efficiently utilized for imaging.

If the transmitted energy cannot be efficiently utilized for imaging as described above, the signal to noise ratio (SNR) of an image as well as the efficiency deteriorates. In order to improve the SNR of an image that gets deteriorated, a technique of increasing light energy to transmit or increasing an attention point or direction of reception to cover the entire transmission range may be considered. However, for example, in the case of irradiating light to the biological object, a maximum allowable illumination is defined due to the safety reason, and thus it is difficult to increase light energy to transmit without limitation. Further, in the case of simply increasing the number of attention points or directions of reception, a processing circuit of a received signal increases in size.

Next, a received acoustic wave and a signal process in the ultrasonic wave echo and the photoacoustic tomography will be described.

In the ultrasonic wave echo, a transmitted ultrasonic wave is reflected from an interface or a scatterer having different acoustic impedance inside the subject. Since the ultrasonic wave gets frequency dependant attenuation while being propagated inside the subject, the ultrasonic wave having the center frequency lower than the frequency of the transmitted ultrasonic wave is received. For example, in imaging the inside of the subject, the ultrasonic wave having the center frequency of about 3 MHz to 5 MHz is received in the case of observing the depth of 10 cm or more, and the ultrasonic wave having the center frequency of about 7 MHz to 15 MHz is received in the case of observing a section that is as shallow as 5 cm or less.

Meanwhile, in the photoacoustic tomography, a photoacoustic wave is generated from an object having a different optical absorption coefficient. If the size of the object is large, the photoacoustic wave having the low center frequency is generated, whereas if the size of the object is small, the photoacoustic wave having the high center frequency is generated. In the case of objects having the same absorption coefficient, if the size of the object is large, the amplitude of the photoacoustic wave to be generated increases, whereas if the size of the object is small, the amplitude of the photoacoustic wave to be generated decreases. That is, since the small object generates the photoacoustic wave that is high in center frequency and small in amplitude, it becomes difficult to propagate up to the acoustic conversion element. For example, in the case of observing the depth of about 5 cm, the photoacoustic wave received by the photoacoustic tomography has the center frequency of 1 MHz to 3 MHz. In the case of observing the deeper section, a signal having a lower center frequency is received. For example, in Non Patent Literature 1, an acoustic conversion element that has center frequency of 1 MHz is employed to observe the depth of about 1 cm to 5 cm. As described above, in the case of imaging the same depth inside the subject by the photoacoustic tomography and the ultrasonic wave echo, the received acoustic waves are greatly different in center frequency.

In the signal process, by performing sampling at a sampling frequency twice as high as a frequency included in a signal to process, original information can be retained. That is, in the case of processing an ultrasonic wave signal that includes a significant signal component up to 20 MHz, the sampling frequency of 40 MHz or more is necessary. For example, in the case of performing an adding process on an input of 64 CH including a significant signal component up to 20 MHz in real time, 63 adding circuits that can perform the adding process at 40 MHz or more and a circuit having a processing ability capable of performing the adding process of 2,520,000,000 times per second are necessary. Further, for example, in the case of processing a photoacoustic signal of 64 CH including a significant signal component up to 3 MHz, 63 adding circuits that can perform the adding process at 6 MHz or more and the adding processing ability of 189,000,000 times per second are necessary. As described above, the adding process ability required at minimum greatly depends on the frequency of the signal. If circuits for adding the ultrasonic wave echo signal having the high center frequency and circuits for adding the photoacoustic signal having the low center frequency are communalized "as is", a circuit is constituted suitable for the ultrasonic wave echo signal in which the high processing ability is required. For this reason, when processing the photoacoustic signal in which even the lower processing ability can be used, the process is performed more than necessary, and therefore, it is inefficient.

The inventor(s) has conducted a study and has found that the efficiency of the adding process circuit deteriorates because the photoacoustic wave signal and the ultrasonic wave echo signal have different frequencies from each other. For this reason, there is a need for providing a subject information processing apparatus (e.g., a biological information processing apparatus) that can execute an efficient process according to a characteristic of a received signal by changing an operation of a processing circuit of digital data.

Further, Patent Literature 1 does not disclose an efficient process according to a characteristic of a received signal in a processing circuit including an adding process circuit in which an adding process is performed by an analog circuit, and thereafter conversion to a digital signal is performed.

In light of the above-mentioned problems, it is a-

nobject of the present invention to provide a technique of suppressing the size of a processing circuit in a subject information processing apparatus which can execute the ultrasonic wave echo and the photoacoustic tomography.

[Solution to Problem]

This invention provides a subject information processing apparatus, comprising:

a plurality of first acoustic conversion elements that receives an acoustic wave having a first frequency that is transmitted from an acoustic wave transmitter and reflected inside a subj ect, and converts the acoustic wave into an analog signal;

a plurality of second acoustic conversion elements that receives an acoustic wave having a second frequency that is generated when light emitted from a light source is irradiated to a subject, and converts the acoustic wave into an analog signal;

an analog-to-digital (A/D) converter that samples the analog signal converted in each of the plurality of first and second acoustic conversion elements and converts the sampled analog signal into a digital signal;

a data memory that retains the digital signal converted by the A/D converter;

a controller that instructs the data memory to output the digital signal at a predetermined sampling frequency for each receiving axis; and

a processor that generates image data based on the digital signal output from the data memory,

wherein the first frequency is set to be higher than second frequency, and

in the digital signal output from the data memory, the number of samples per unit time of the digital signal derived from the acoustic wave having the second frequency on one receiving axis is smaller than the number of samples per unit time of the digital signal derived from the acoustic wave having the first frequency on one receiving axis.

[Advantageous Effects of Invention]

According to the present invention, in the subject information processing apparatus which can execute the ultrasonic wave echo and the photoacoustic tomography, the size of the processing circuit can be suppressed.

Further features of the present invention will become apparent from the following description of exemplary embodiments with reference to the attached drawings.

[Brief Description Of Drawings]

[Fig. 1]

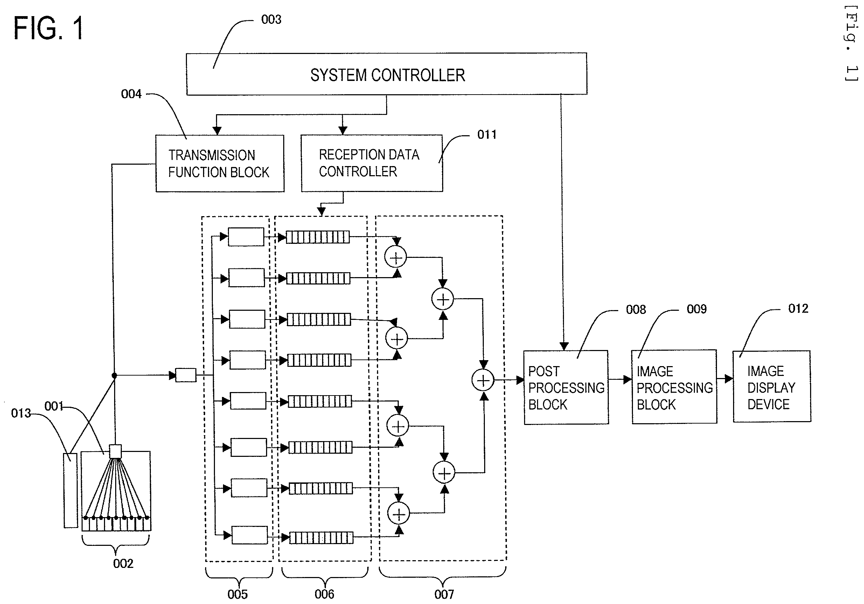

Fig. 1 is a system overview diagram of a first exemplary embodiment.
[Fig. 2]

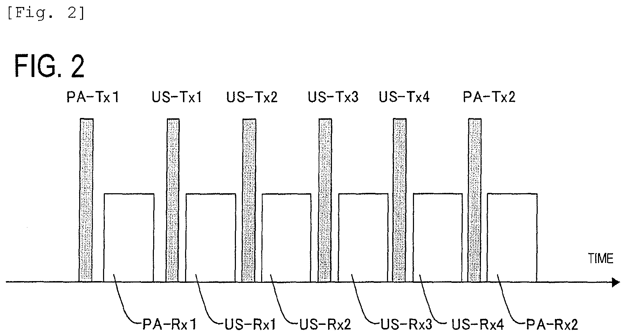

Fig. 2 is a diagram illustrating a processing timing of the first exemplary embodiment.
[Fig. 3]

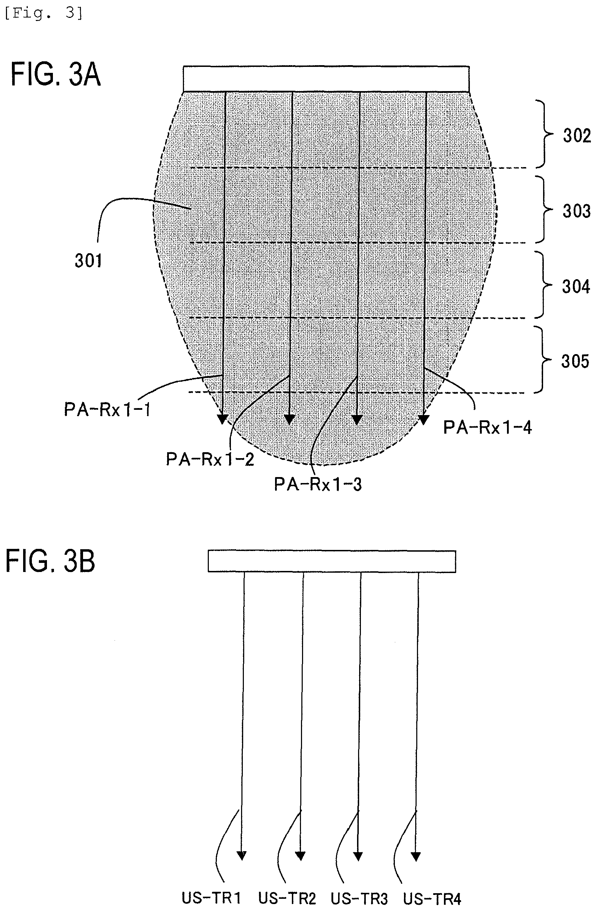

Fig. 3 illustrates diagrams illustrating an observation area of the first exemplary embodiment.
[Fig. 4]

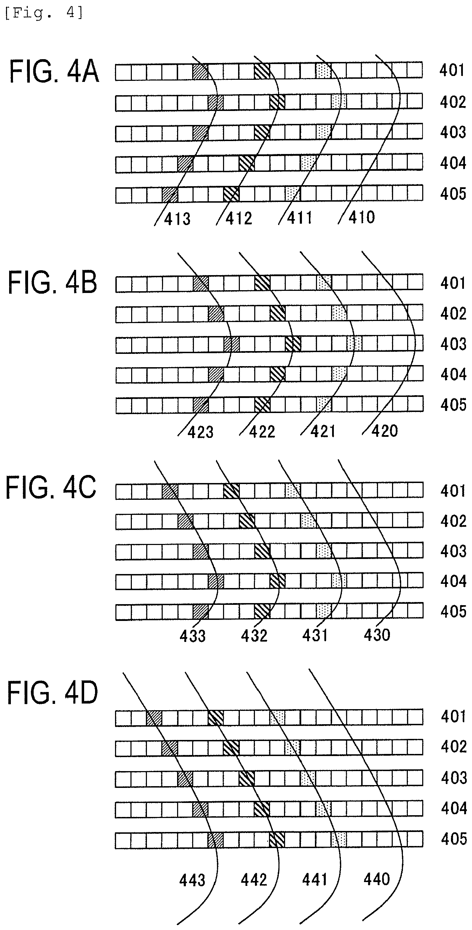

Fig. 4 illustrates diagrams for explaining a data read location of the first exemplary embodiment.
[Fig. 5]

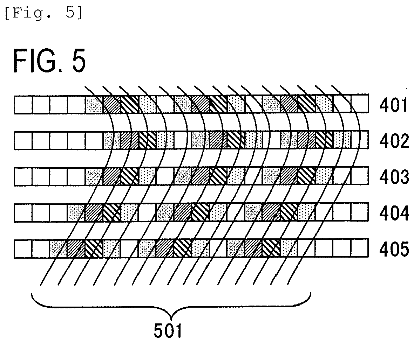

Fig. 5 is a diagram for explaining a data read location of the first exemplary embodiment.
[Fig. 6]

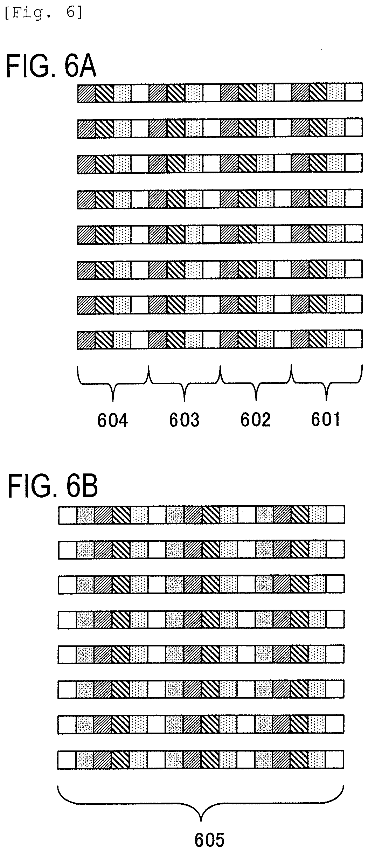

Fig. 6 illustrates diagrams for explaining non-added data of the first exemplary embodiment.
[Fig. 7]

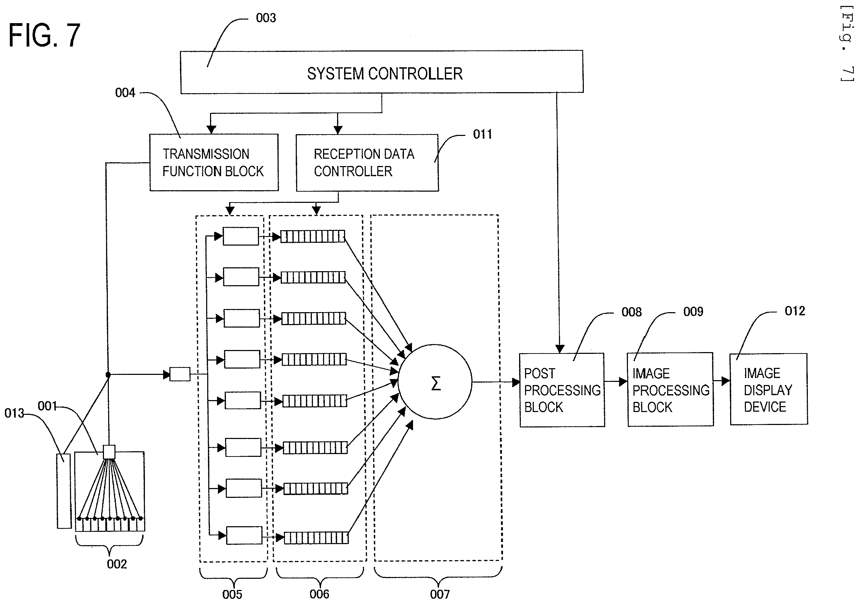

Fig. 7 is a system overview diagram of a second exemplary embodiment.
[Fig. 8]

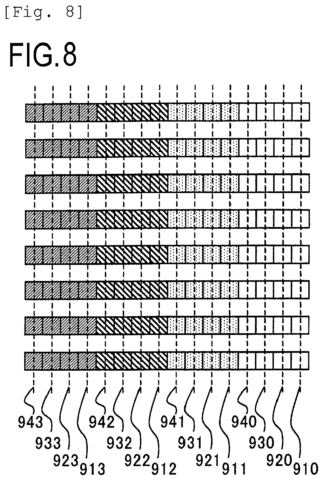

Fig. 8 is a diagram for explaining non-added data of the second exemplary embodiment.
[Fig. 9]

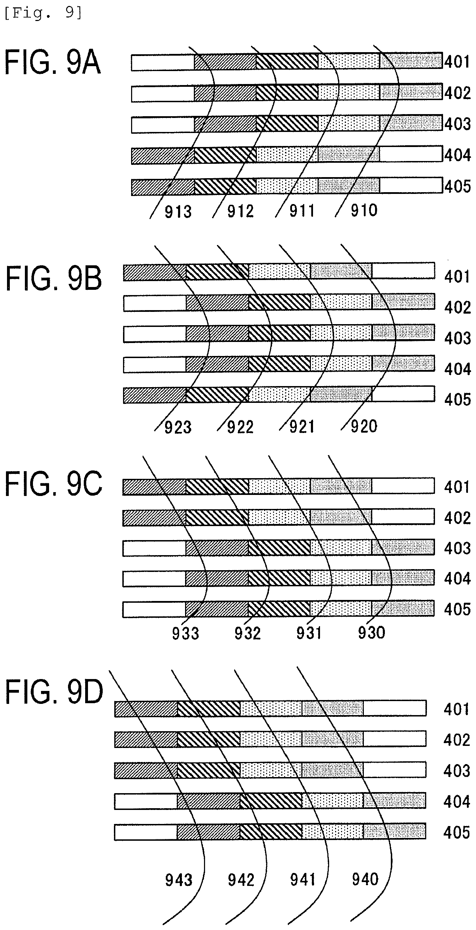

Fig. 9 illustrates diagrams for explaining a data read location of the second exemplary embodiment.
[Fig. 10]

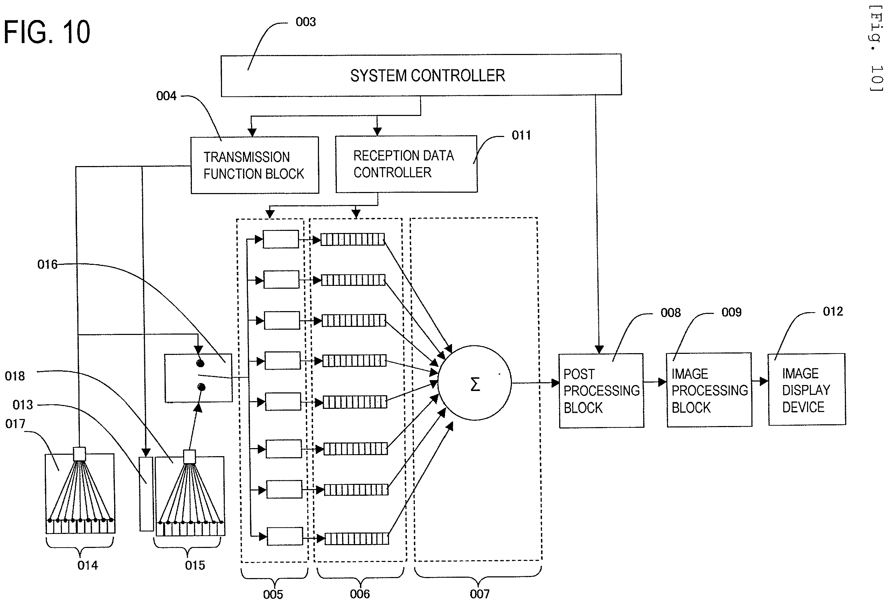

Fig. 10 is a system overview diagram of a third exemplary embodiment.
[Fig. 11]

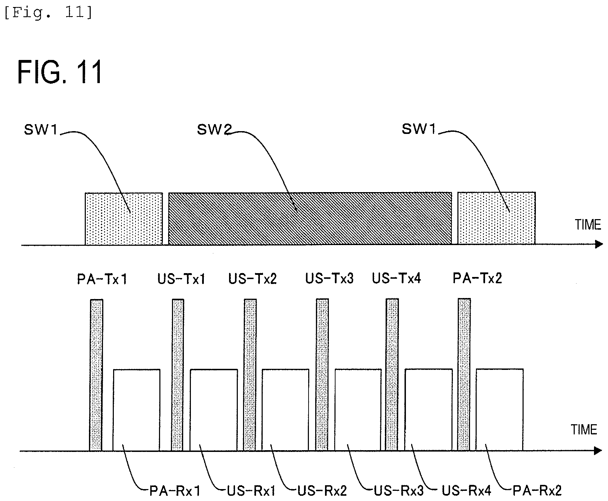

Fig. 11 is a diagram illustrating a timing of a switch of the third exemplary embodiment.

[Description of Embodiments]

Hereinafter, exemplary embodiments of a subject information processing apparatus according to the present invention will be described in detail. Further, in the present invention, an "acoustic wave" includes ones which are called a sonic wave, an ultrasonic wave, and a photoacoustic wave. In the following description, a "photoacoustic wave" refers to an acoustic wave generated inside a subject by irradiating light to the inside of the subject using the photoacoustic tomography, and an "ultrasonic wave" refers to an acoustic wave that is transmitted to the subjected and received from the subject by an acoustic conversion element by using the an ultrasonic wave echo. A biological information processing apparatus will be described below as a typical example of a subject information processing apparatus.

(First Exemplary Embodiment)

Fig. 1 is a diagram illustrating a system configuration of a biological information processing apparatus according to a first exemplary embodiment of the present invention.

The present system includes a probe 001 including a plurality of acoustic conversion elements 002, a system controller 003, a transmission function block 004, an analog-to-digital (A/D) conversion block 005, and a data memory block 006. The present system further includes an adding block 007, a post processing block 008, an image processing block 009, an image display device 012, a reception data controller 011, and a light source 013.

First, a timing of performing the photoacoustic tomography and the ultrasonic wave echo will be described with reference to Fig. 2. First light irradiation is performed at a timing represented by PA-Tx1, and a photoacoustic wave emitted from the subject is received and processed during a time period PA-Rxl. Next, an ultrasonic wave is transmitted to the subject at timings represented by US-Tx1 to US-Tx4, and received and processed during time periods US-Rx1 to US-Rx4, respectively. Further, the next light irradiation is performed at a timing represented by PA-Tx2, and the above-described process is repeated.

Fig. 3 illustrates diagrams schematically illustrating a range, inside the subject, acquired by one time light irradiation and the ultrasonic wave echo of multiple times. Fig. 3A illustrates that imaging is performed on an area 301 to which light is irradiated at PA-Tx1 of Fig. 2 in a direction indicated by arrows of receiving axes represented by PA-Rx1-1 to PA-Rx1-4. The receiving axis refers to a locus of a point on a spatial axis on which image reconstruction is performed at the time of image reconstruction (generation of image data) for generating pixel data or voxel data and represents a locus of an attention point that moves in a depth direction of the subject with respect to the position of the acoustic conversion element in Fig. 3. Further, for convenience, the area 301 may be divided into areas 302 to 305 that are in an order close to the probe 001. Fig. 3B illustrates that the US-TR1 is used as the receiving axis of the ultrasonic wave received, during the time period US-Rx1, corresponding to the ultrasonic wave transmitted at US-Tx1 of Fig. 2, and imaging is performed by transmission and reception of the ultrasonic wave of four times of the receiving axes from US-TR1 to US-TR4. The area illustrated in Fig. 3A has the same width as the area illustrated in Fig. 3B. Transmission in the ultrasonic wave echo can be converged by transmitting the ultrasonic wave having a shifted timing from the acoustic conversion element. However, since it is difficult to converge light inside the strong scatterer, the light irradiation area 301 have the width including a plurality of photoacoustic signal receiving axes. Further, in the present embodiment, it is assumed that in a received signal obtained by the ultrasonic wave echo, a significant signal is included up to FusHz, and in the photoacoustic wave obtained by the photoacoustic tomography, a significant signal is included up to $F_{PA}$Hz. As an example, the present embodiment will be described in connection with a case where $F_{US}$ is 20 MHz, and $F_{PA}$ is 5 MHz. However, effects of the present invention can be obtained as long as a condition of $F_{us} > F_{PA}$ is satisfied.

In the present embodiment, the acoustic conversion element is an ultrasonic wave transmitter (an acoustic wave transmitter) and also functions as both a first acoustic conversion element (an acoustic conversion element that receives an ultrasonic wave) and a second acoustic conversion element (an acoustic conversion element that receives a photoacoustic wave). The first acoustic conversion element and the second acoustic conversion element may be separately prepared, and the present invention is not limited to the above-described example. Further, a frequency of the ultrasonic wave transmitted by the ultrasonic wave echo is a first frequency, and a frequency of the photoacoustic wave is a second frequency. The A/D conversion block corresponds to an A/D converter of the present invention, and the data memory block corresponds to a data memory. Further, the adding block, the post processing block, and the image processing block correspond to a processor that generates image data, and the reception data controller corresponds to a controller.

Here, returning back to Fig. 1, an operation when performing the ultrasonic wave echo will be described in connection with transmission at a timing represented by US-Tx1 of Fig. 2 and an operation during a time period represented by US-Rx1 as an example.

First, a transmission trigger is output from the system controller 003 to the transmission function block 004. The transmission function block 004 calculates a delay time to be conferred to transmission for each of the acoustic conversion elements by using the distance from each acoustic conversion element with respect to a focal position in a direction US-TR1 for transmitting the ultrasonic wave and the velocity of sound inside the subject. A voltage signal that is an electrical signal shifted by the delay time is transmitted to each acoustic conversion element 002. Further, in the ultrasonic wave echo, a transmission beam for transmitting the ultrasonic wave or a reception beam of the reflected ultrasonic wave is consistent with the receiving axis. For example, the receiving axis represented by US-TR1 of Fig. 3B corresponds to the transmission beam and the reception beam.

The acoustic conversion element 002 converts the input voltage signal into an ultrasonic wave and transmits the ultrasonic wave to the inside of the subject. The ultrasonic wave reflected inside the subject is received by the acoustic conversion element 002, converted into an analog electrical signal (an analog signal), and input to the A/D conversion block 005. The input analog electrical signal that is derived from the ultrasonic wave is converted into digital data (a digital signal) and transmitted to the data memory block 006. The data memory block 006 retains the input digital data that is derived from the ultrasonic wave in a memory disposed therein.

The reception data controller 011 outputs a data read location inside the memory to the data memory block 006 based on information related to the receiving axis US-TR1 instructed from the system controller 003. The data memory block 006 outputs data, instructed from the reception data controller 011, among retained digital data to the adding block 007 as non-added data.

Here, data reading inside the memory will be described with reference to Fig. 5. Fig. 5 schematically illustrates memories 401 to 405 in the data memory block 006 that correspond to the acoustic conversion elements, respectively. In the adding block 007 of Fig. 1, 8 CH are present as an input, but for simplification, it is here illustrated that memories corresponding to 5 CH are disposed inside the data memory block 006. Further, digital data that is early in reception time is retained in the right of the drawing. In the drawing, data, which are instructed to output at the same timing, among non-added data instructed from the reception data controller 011 are expressed by the same color and connected by lines 501.

In the ultrasonic wave echo of the present embodiment, since the significant signal component is included up to 20 MHz ($F_{US}$), the sampling frequency of 40 MHz (2 * Fus) or more becomes necessary. The sampling frequency of 40 MHz corresponds to a segment, 25 nanoseconds (1/(2 * $F_{US}$), of the memories 401 to 405 in a time direction. Data to be output from the data memory block 006 needs to be continuously read from each of the memories 401 to 405 in the time direction without skipping. As a result, in the present embodiment, the number of pieces of data per unit time of the non-added data on the receiving axis US-TR1 is the same value as the sampling frequency, that is, 40,000,000.

Returning back to Fig. 1, the adding block 007 multiplies the weight by each input non-added data according to a need, adds all of the input non-added data, and outputs the added data to the post processing block 008. The post processing block computes an envelope curve of the input data and outputs the result to the image processing block 009. The image processing block 009 performs various imaging processes such as a rearrangement or smoothing of data appropriate for an observation area or edge emphasis on the input data and transmits the image processing result to the image display device 012 as brightness value data. Finally, an image is displayed on the image display device 012.

Next, an operation when performing the photoacoustic tomography will be described in connection with transmission at a timing represented by PA-Tx1 of Fig. 2 and an operation during a time period represented by PA-Rx1 as an example. First, a transmission trigger is output from the system controller 003 to the transmission function block 004. The transmission function block generates a signal for driving the light source 013, and so the light source 013 transmits light energy to the subject. The light energy is spread to the area 301. A photoacoustic wave generated by the light energy is received by the plurality of acoustic conversion elements 002, converted into an analog electrical signal, and then input to the A/D conversion block 005. The A/D conversion block 005 converts the input analog electrical signal derived from the photoacoustic wave into digital data and transmits the digital data to the data memory block 006. The data memory block 006 retains the input digital data derived from the photoacoustic wave in a memory disposed therein.

The reception data controller 011 outputs a data read location inside the memory to the data memory block 006 based on information related to the receiving axes PA-Rx1-1, PA-Rx1-2, PA-Rx1-3, and PA-Rx1-4 instructed from the system controller 003. The data memory block 006 outputs data, instructed from the reception data controller 011, among retained digital data to the adding block 007 as non-added data.

Here, data reading inside the memory will be described with reference to Fig. 4. Fig. 4 schematically illustrates the memories 401 to 405 in the data memory block 006 that correspond to the individual acoustic conversion elements, respectively. Further, digital data that is early in reception time is retained in the right of the drawing. Fig. 4A corresponds to the receiving axis PA-Rx1-1, Fig. 4B corresponds to the receiving axis PA-Rx1-2, Fig. 4C corresponds to the receiving axis PA-Rx1-3, and Fig. 4D corresponds to the receiving axis PA-Rx1-4. In drawings, data, which are output at the same timing (time), among non-added data instructed from the reception data controller 011 are expressed by the same color, and data output at the same time are connected by lines as connected by a line 410.

In the present embodiment, since the photoacoustic wave received by the photoacoustic tomography includes the significant signal component up to 5 MHz ($F_{PA}$), the sampling frequency of 10 MHz (2 * $F_{PA}$) or more becomes necessary. The present system performs sampling of 40 MHz (2 * $F_{US}$) suitable for the received signal of the ultrasonic wave echo. For this reason, if three of four samples are skipped in the time direction inside the memories 401 to 405 and read as non-added data, processing can be performed without losing information of the signal received by the photoacoustic tomography. For this reason, for the receiving axis of PA-Rx1-1, as illustrated in lines 410 to 413, even if data in which three of four consecutive samples are skipped are read as the non-added data, processing can be performed without losing information received by the photoacoustic tomography. Similarly, for the receiving axis of PA-Rx1-2, as illustrated in lines 420 to 423, data inside the memories is read in units of four samples as the non-added data. Further, for the receiving axis of PA-Rx1-3, as illustrated in lines 430 to 433, data inside the memories is read in units of four samples as the non-added data. Further, for the receiving axis of PA-Rx1-4, as illustrated in lines 440 to 443, data inside the memories is read in units of four samples as the non-added data.

As described above, in the process of the photoacoustic tomography, by reading out data while skipping in the time direction, the number of pieces of data of the non-added data per unit time on each receiving axis becomes smaller than the sampling frequency. That is, the number of pieces of data can become smaller than the number of pieces of data per unit time on each receiving axis in the ultrasonic wave echo.

Next, the adding block 007 multiplies the weight by each input non-added data, adds all of the input non-added data, and outputs the added data to the post processing block 008. The post processing block 008 computes an envelope curve of the input data or performs a calculation expressed by the following Equation 1 and outputs the result to the image processing block 009. Further, $S_{in}$ denotes input data, $S_{out}$ denotes an output, t denotes a time elapsed since reception of the photoacoustic wave started.

[Math. 1]

$$S_{out}(t) = 2S_{in}(t) - 2t\frac{\partial S_{in}(t)}{\partial t}$$

The image processing block 009 performs various imaging processes such as a rearrangement or smoothing of data appropriate for an observation area or edge emphasis on the input data and transmits the image processing result to the image display device 012 as brightness value data. Finally, an image is displayed on the image display device 012.

As described above, by setting a plurality of receiving axes in the light irradiation area 301 and performing imaging, imaging of the inside of the subject can be performed using efficiently irradiated light energy. Therefore, it is possible to suppress the SNR of an image from deteriorating due to deterioration of the use efficiency of the light energy.

Next, the non-added data in the ultrasonic wave echo and the photoacoustic tomography will be described with reference to Fig. 6. Fig. 6B illustrates non-added data 605 of the signal received by the ultrasonic wave echo. The non-added data present in the right of the drawing is data that is early output from the data memory block 006. Eight segments of the non-added data 605 in an up-down direction represent an input of 8 CH to the adding block 007 in Fig. 1. The adding block 007 of the present embodiment is designed to process data of 8 CH as an input for every 40 MHz. That is, the non-added data 605 in the drawing is input "as is" for every 40 MHz. That is, the number of samples per unit time on the receiving axis US-TR1 is 40,000,000.

Fig. 6A illustrates non-added data of the signal received by the photoacoustic tomography. Non-added data 601 is non-added data related to the receiving axis PA-Rx1-1. Similarly, non-added data 602 related to the receiving axis PA-Rx1-2, non-added data 603 related to the receiving axis PA-Rx1-3, and non-added data 604 related to the receiving axis PA-Rx1-4 are illustrated. As described above with reference to Fig. 5, in each receiving axis, the non-added data related to each receiving axis is data read from the memory in units of four samples, that is, data sampled at 10 MHz.

As described above, in the reception process of the ultrasonic wave echo, the number of pieces of data per unit time of the non-added data on the receiving axis US-TR1 is 40,000,000. Further, in the reception process of the photoacoustic tomography, the number of pieces of data per unit time of the non-added data on the receiving axes PA-Rx1-1 to PA-Rx1-4 is 10,000,000. Therefore, the reception process of the photoacoustic tomography is smaller in number of data per unit time than the reception process of the ultrasonic wave echo.

Here, in both the ultrasonic wave echo and the photoacoustic tomography, data of 8 CH are sequentially input to the adding block 007 at 40 MHz. As a result, the processing circuit of the small size using efficiently the adding ability as well as commnonalization of a circuit including the adding block 007 can be implemented.

By performing the configuration and operation of the system, the light energy irradiated to the light irradiation area 301 can be efficiently used for imaging, and the adding ability of the adding block 007 can be efficiently used. As a result, the SNR of an image does not deteriorate, and the ultrasonic wave echo and the photoacoustic tomography can be processed in real time by a common circuit. Further, the system of the small-sized processing circuit can be obtained.

Further, in the present embodiment, the adding block having the input of 8 CH has been described, but the present invention is not limited thereto. For example, an input of multiple CH such as 64 CH, 128 CH, and 256 CH may be implemented.

Further, in the present embodiment, transmission and reception in the ultrasonic wave echo have been described so that the receiving axis becomes one direction on one time transmission, but the present invention is not limited thereto. In the case of a circuit having the size capable of implementing the receiving axis process of N directions on one time transmission of the ultrasonic wave echo, the receiving axis of the photoacoustic tomography process can increase by N times, and the same effects can be obtained.

Further, in the present embodiment, an example of a linear scan has been described, but even in the case of a scan technique such as a sector scan or a convex scan, the effects can be obtained by the same processing.

(Second Exemplary Embodiment)

Fig. 7 is a diagram schematically illustrating a system configuration of a biological information processing apparatus according to a second exemplary embodiment of the present invention.

The system diagram of Fig. 7 is different from the system schematic diagram (Fig. 1) of the first exemplary embodiment of the present system in that the reception data controller 011 is connected even to the A/D conversion block 005. Further, the adding block 007 is a circuit having an adding ability and is expressed by a symbol $\Sigma$.

Timing for performing the photoacoustic tomography and the ultrasonic wave echo is the same as in the first exemplary embodiment illustrated in Fig. 2. Even in the present embodiment, it is assumed that in the received signal obtained by the ultrasonic wave echo, the significant signal is included up to $F_{US}$Hz, and in the photoacoustic wave obtained by the photoacoustic tomography, the significant signal is included up to $F_{PA}$Hz. As an example, the present embodiment will be described in connection with a case in which $F_{US}$ is 20 MHz, and $F_{PA}$ is 5 MHz, but the effects of the present invention can be obtained as long as a condition of $F_{US} > F_{PA}$ is satisfied.

A range inside the subject acquired one time light irradiation and the ultrasonic wave echo of multiple times is the same as illustrated in Fig. 3. The reference numerals 302 to 305 in Fig. 3 represent a range of the depth.

A system operation at the time of imaging by the ultrasonic wave echo is the same as in the first exemplary embodiment, and a description thereof will not be repeated.

Next, an operation when performing the photoacoustic tomography will be described. First, before transmitting the light energy to the subject, the sampling frequency of the A/D conversion block 005 is changed by an instruction from the reception data controller 011. In the present embodiment, 10 MHz ($2 * F_{PA}$) is described as the changed sampling frequency. However, if the changed sampling frequency is lower than the sampling frequency at the time of acquiring the ultrasonic wave echo and higher than the frequency twice as high as the significant signal included in the photoacoustic wave, the effects of the present invention are obtained.

Thereafter, the light energy is transmitted from the light source 013 to the subject. The generated photoacoustic wave is received by the plurality of acoustic conversion elements 002. The plurality of acoustic conversion elements 002 converts the photoacoustic wave into an analog electrical signal and then inputs the analog electrical signal to the A/D conversion block 005. The A/D conversion block 005 converts the input analog electrical signal into digital data with the changed sampling frequency of 10 MHz and transmits the digital data to the data memory block 006. The data memory block 006 retains the input digital data in a memory disposed therein.

The reception data controller 011 outputs a data read location inside the memory to the data memory block 006 based on information related to the receiving axes PA-Rx1-1, PA-Rx1-2, PA-Rx1-3, and PA-Rx1-4 instructed from the system controller 003. The data memory block 006 outputs data, instructed from the reception data controller 011, among retained digital data to the adding block 007 as non-added data.

Here, data reading inside the memory will be described with reference to Fig. 9. Fig. 9 schematically illustrates the memories 401 to 405 in the data memory block 006 that correspond to the individual acoustic conversion elements, respectively. Further, digital data that is early in reception time is retained in the right of the drawing. In the present embodiment, since sampling is performed at 10 MHz, a signal is retained at every 100 nanoseconds. Fig. 9A corresponds to the receiving axis PA-Rx1-1, Fig. 9B corresponds to the receiving axis PA-Rx1-2, Fig. 9C corresponds to the receiving axis PA-Rx1-3, and Fig. 9D corresponds to the receiving axis PA-Rx1-4. In drawings, data, which are output at the same timing (time), among non-added data instructed from the reception data controller 011 are expressed by the same color and are connected by lines 910 to 913, lines 920 to 923, lines 930 to 933, and lines 940 to 943. In the present embodiment, since the photoacoustic wave received by the photoacoustic tomography includes the significant signal component up to 5 MHz ($F_{PA}$), the sampling frequency of 10 MHz ($2 * F_{PA}$) or more becomes necessary. In the present embodiment, as described above, sampling is performed at 10 MHz ($2 * F_{PA}$) in the A/D conversion block 005. Therefore, even if it is output as the non-added data "as is" without skipping in the time axis direction, data of a plurality of receiving axes can be processed without losing information.

Next, the non-added data will be described with reference to Fig. 8. Fig. 8 illustrates non-added data of the received signal in the photoacoustic tomography. The non-added data present in the right of the drawing is data that is early output from the data memory block 006. In Fig. 9, line numbers 910 to 943 connecting the non-added data that is output at the same time and line represented by dotted lines in Fig. 8 represent the same data. That is, unlike a technique of outputting data of the next receiving axis after outputting all of data of one receiving axis, data is transmitted while sequentially changing the receiving axis.

A detailed description will be made with reference to Fig. 3. In the first exemplary embodiment, after data of the receiving axis PA-Rx1-1 is output as the non-added data, data of the receiving axis PA-Rx1-2 is transmitted. However, in the present embodiment, data of a reception area of the depth 302 of the receiving axis PA-Rx1-1 is first output. Subsequently, data of the reception area of the depth 302 of the receiving axis PA-Rx1-2, data of the reception area of the depth 302 of the receiving axis PA-Rx1-3, and data of the reception area of the depth 302 of the receiving axis PA-Rx1-4 are sequentially output. Then, on the next depth, the non-added data is sequentially output starting from data of the reception area of the depth 303 of the receiving axis PA-Rx1-1. The non-added data is sequentially output starting from digital data related on an area closest to the acoustic conversion element among the areas inside the subject. The reception area refers to a range defined by a predetermined distance range (a predetermined distance range in a direction from the acoustic conversion element to the subject depth) in a certain receiving axis.

In the case of outputting the non-added data in the above-described order, after outputting the non-added data related to the depth 302, digital data related to the depth 302 becomes unnecessary. Therefore, after outputting the non-added data related to the depth 302, new digital data can be immediately written in the memory area. By performing such an operation, the memory size necessary for the system can be controlled, and thus a biological information processing apparatus can be provided at a lower cost.

An operation after inputting the output non-added data to the adding block 007 is the same as in the first exemplary embodiment, and a description thereof will not be repeated.

By changing the sampling frequency of the A/D conversion block 005 as described above, the number of pieces of data per unit time at the time of the photoacoustic tomography process can be smaller than the number of pieces of data per unit time at the time of the ultrasonic wave echo process. Further, in both the ultrasonic wave echo and the photoacoustic tomography, as data input to the adding block 007, data of 8 CH are sequentially input at 40 MHz. As a result, not only commnonalization of the circuit including the adding block 007 but also the small-sized processing circuit using efficiently the adding ability can be implemented.

In the present embodiment, by lowering the operational frequency of the A/D conversion block, the noise that causes heat generation of an ADC (an analog-to-digital converter) is reduced, and power consumption of the system is reduced. That is, a biological information processing apparatus that is high in SNR of an image and low in power consumption can be obtained.

(Third Exemplary Embodiment)

Fig. 10 is a diagram illustrating a system configuration of a biological information processing apparatus according to a third exemplary embodiment of the present invention.

The present system includes a first probe 017 including a plurality of first acoustic conversion elements 014, a second probe 018 including a plurality of second acoustic conversion elements 015, a system controller 003, a transmission function block 004, and an analog-to-digital (A/D) conversion block 005. The present system further includes a data memory block 006, an adding block 007, a post processing block 008, an image processing block 009, an image display device 012, a reception data controller 011, a light source 013, and a switch 016.

The third exemplary embodiment is the same as in the above-described exemplary embodiments except that two kinds of probes are disposed, and a switch is added.

As described above, the center frequency of the photoacoustic wave in the photoacoustic tomography is different from the center frequency of the reflected ultrasonic wave in the ultrasonic wave echo. Further, the acoustic conversion element also has a receivable bandwidth and a center frequency characteristic. For this reason, an optimum acoustic conversion element used in the photoacoustic tomography may be different from an optimum acoustic conversion element used in the ultrasonic wave echo. For example, an acoustic conversion element using a PZT may have a center frequency of 1 MHz or 20 MHz, and a fractional bandwidth of about 80% is implemented. Further, a CMUT using a semiconductor technology may have a fractional bandwidth exceeding 100%. A combination of the acoustic conversion element used in the photoacoustic tomography and the acoustic conversion element used in the ultrasonic wave echo may depend on the observation depth of the target subject or the desired spatial resolution.

Particularly, the center frequency of the first acoustic conversion element that receives the reflected ultrasonic wave by the ultrasonic wave echo is preferably higher than the center frequency of the second acoustic conversion element that receives the photoacoustic wave by the photoacoustic tomography. In this case, the ultrasonic wave can be received at high efficiency.

Therefore, the acoustic conversion element 014 of the present embodiment has a center frequency characteristic higher than the acoustic conversion element 015. For example, the acoustic conversion element 014 has the center frequency of 12 MHz, and the acoustic conversion element 015 has the center frequency of 3 MHz.

Since a signal processing flow of the present embodiment is the same as in the above-described exemplary embodiments, a description thereof will not be repeated, and only an operation different from the above-described exemplary embodiments will be described.

The switch 016 has a function of switching a connection between the acoustic conversion elements 014 and 015 and the A/D conversion block and can connect any one of the acoustic conversion elements with the A/D conversion block. In the case of performing the photoacoustic tomography, the switch 016 operates to cause a connection with the acoustic conversion element 015, and in the case of performing the ultrasonic wave echo, the switch 017 operates to cause a connection with the acoustic conversion element 014. Fig. 11 is a diagram illustrating operation timing. In the case of transmitting light energy at a timing PA-Tx1, a state (SW1) switched to the acoustic conversion element 015 is maintained. Thereafter, during a time period from US-Tx1 to US-Tx4 in which the ultrasonic wave echo is performed, the switch 016 operates to maintain a state (SW2) switched to the acoustic conversion element 14.

By performing the above-described operation, a signal received in each of the photoacoustic wave and the ultrasonic wave echo can be more efficiently converted into an analog electrical signal. Therefore, a biological information processing apparatus that has the higher SNR and an improved image quality can be provided.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

This application claims the benefit of Japanese Patent ApplicationNo. 2010-031171, filed on February 16, 2010, which is hereby incorporated by reference herein in its entirety.

This application is a divisional application of European patent application no. 11 706 365.1 (the "parent application"), also published as EP 2 536 338. Based on the original claims of the parent application, the following aspects form part of the content of this divisional application as filed.

1. A subject information processing apparatus, comprising:

a plurality of first acoustic conversion elements that receives an acoustic wave having a first frequency that is transmitted from an acoustic wave transmitter and reflected inside a subject, and converts the acoustic wave into an analog signal;

a plurality of second acoustic conversion elements that receives an acoustic wave having a second frequency that is generated when light emitted from a light source is irradiated to the subject, and converts the acoustic wave into an analog signal;

an A/D converter that samples the analog signal converted in each of the plurality of first and second acoustic conversion elements and converts the sampled analog signal into a digital signal;

a data memory that retains the digital signal converted by the A/D converter;

a controller that instructs the data memory to output the digital signal at a predetermined sampling frequency for each receiving axis; and

a processor that generates image data based on the digital signal output from the data memory,

wherein the first frequency is set to be higher than the second frequency, and

in the digital signal output from the data memory, the number of samples per unit time of the digital signal derived from the acoustic wave having the second frequency on one receiving axis is smaller than the number of samples per unit time of the digital signal derived from the acoustic wave having the first frequency on one receiving axis.

2. The subject information processing apparatus according to aspect 1,

wherein the plurality of first acoustic conversion elements has a function of the acoustic wave transmitter.

3. The subject information processing apparatus according to aspect 1 or 2,

wherein the plurality of first acoustic conversion elements has a function of the plurality of second acoustic conversion elements.

4. Thesubject information processing apparatus according to any one of aspects 1 to 3,

wherein the A/D converter converts the analog signal derived from the acoustic wave having the second frequency and the analog signal derived from the acoustic wave having the first frequency into digital signals at the same sampling frequency, and

when the controller instructs the data memory to output, by instructing to output the digital signal derived from the acoustic wave having the second frequency more sparsely than the digital signal derived from the acoustic wave having the first frequency, in the digital signal output from the data memory, the number of samples per unit time of the digital signal derived from the acoustic wave having the second frequency on one receiving axis is smaller than the number of samples per unit time of the digital signal derived from the acoustic wave having the first frequency on one receiving axis.

5. Thesubject information processing apparatus according to any one of aspects 1 to 3,

wherein the A/D converter converts the analog signal derived from the acoustic wave having the second frequency into the digital signal at a lower sampling frequency than the analog signal derived from the acoustic wave having the first frequency, so that in the digital signal output from the data memory, the number of samples per unit time of the digital signal derived from the acoustic wave having the second frequency on one receiving axis is smaller than the number of samples per unit time of the digital signal derived from the acoustic wave having the first frequency on one receiving axis.

6. Thesubject information processing apparatus according to aspect 1 or 2, further comprising:

a switch that performs switching to perform reception using the plurality of first acoustic conversion elements during a time period in which the acoustic wave having the first frequency is received and to perform reception using the plurality of second acoustic conversion elements during a time period in which the acoustic wave having the second frequency is received.

7. Thesubject information processing apparatus according to any one of aspects 1 to 6,
wherein the controller sequentially changes the receiving axis for each reception area defined by a distance from the acoustic conversion element and outputs the digital signal from the memory.

**Claims**

1. A subject information processing apparatus, comprising:

   a plurality of first acoustic conversion elements (002, 014), each of the plurality of first acoustic conversion elements (002, 014) being adapted to receive an acoustic wave having a first frequency that is transmitted from an acoustic wave transmitter and reflected inside a subject, and convert the acoustic wave into a first analog signal;
   a plurality of second acoustic conversion elements (002, 015), each of the plurality of second acoustic conversion elements (002, 015) being adapted to receive an acoustic wave having a second frequency that is generated when light emitted from a light source is irradiated to the subject, and convert the acoustic wave into a second analog signal;
   an A/D converter (005) adapted to convert the first and second analog signal into a first and second digital signal;
   a data memory (006) adapted to retain the first and second digital signal converted by the A/D converter (005) ; and
   a processor (009) adapted to generate image data based on the digital signal output from the data memory,
   wherein the first frequency is higher than the second frequency,

   **characterized in that**
   the A/D converter (005) comprises channels, each of the channels being inputted the first and second analog signals, and
   the A/D converter (005) is adapted to convert the second analog signal into the second digital signal at a lower sampling frequency than a sampling frequency of the first digital signal so that the number of samples per unit time of the second digital signal on one spatial axis on the image data is smaller than the number of the samples per unit time of the first digital signal on one spatial axis on the image data.

2. The subject information processing apparatus according to claim 1,

   wherein the plurality of first acoustic conversion elements has a function of the acoustic wave transmitter.

3. The subject information processing apparatus according to claim 1 or 2,

   wherein the plurality of first acoustic conversion elements has a function of the plurality of second acoustic conversion elements.

4. The subject information processing apparatus according to claim 1 or 2, further comprising:

   a switch adapted to perform switching to perform reception using the plurality of first acoustic conversion elements during a time period in which the acoustic wave having the first frequency is received and to perform reception using the plurality of second acoustic conversion elements during a time period in which the acoustic wave having the second frequency is received.

5. The subject information processing apparatus according to any one of claims 1 to 4, further comprising:

   a controller adapted to sequentially change the spatial axis on the image data for each reception area defined by a distance from the acoustic conversion element and output the digital signal from the memory.

6. The subject information processing apparatus according to claim 1, further comprising:

   a controller (011) adapted to instruct the A/D converter (005) to convert the first and second analog signal into the first and second digital signal at a predetermined sampling frequency or instruct the data memory (006) about a data read location inside the data memory (006).

[Fig. 1]

FIG. 1

SYSTEM CONTROLLER 003

TRANSMISSION FUNCTION BLOCK 004

RECEPTION DATA CONTROLLER 011

005

006

007

POST PROCESSING BLOCK 008

IMAGE PROCESSING BLOCK 009

IMAGE DISPLAY DEVICE 012

001

002

013

[Fig. 2]

# FIG. 2

PA-Tx1    US-Tx1    US-Tx2    US-Tx3    US-Tx4    PA-Tx2

TIME

PA-Rx1   US-Rx1   US-Rx2   US-Rx3  US-Rx4   PA-Rx2

[Fig. 3]

## FIG. 3A

302
303
304
305

301

PA-Rx1-1
PA-Rx1-2
PA-Rx1-3
PA-Rx1-4

## FIG. 3B

US-TR1   US-TR2   US-TR3   US-TR4

[Fig. 4]

FIG. 4A

401
402
403
404
405

413  412  411  410

FIG. 4B

401
402
403
404
405

423  422  421  420

FIG. 4C

401
402
403
404
405

433  432  431  430

FIG. 4D

401
402
403
404
405

443  442  441  440

[Fig. 5]

# FIG. 5

401

402

403

404

405

501

[Fig. 6]

# FIG. 6A

604     603     602     601

# FIG. 6B

605

[Fig. 7]

FIG. 7

[Fig. 8]

# FIG.8

[Fig. 9]

## FIG. 9A

913 912 911 910

## FIG. 9B

923 922 921 920

## FIG. 9C

933 932 931 930

## FIG. 9D

943 942 941 940

# FIG. 10

EP 3 199 973 A1

[Fig. 10]

[Fig. 11]

# FIG. 11

Europäisches
Patentamt

European
Patent Office

Office européen
des brevets

# EUROPEAN SEARCH REPORT

Application Number

EP 17 15 5970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/154298 A1 (CANON KK [JP]; SOMEDA YASUHIRO [JP]; SAITO KEISHI [JP]; ASAO YASUFUMI) 23 December 2009 (2009-12-23)<br>* abstract; figures 1B, 2 *<br>* page 10, line 16 - page 11, line 16 *<br>* page 14, line 24 - page 16, line 8 *<br>* page 17, line 14 - line 22 *<br>* page 18, line 8 - line 14 *<br>* page 24, line 14 - line 16 *<br>* page 33, line 14 - line 17 *<br>----- | 1-6 | INV.<br>G01S7/52<br>A61B5/00<br>A61B8/13<br>G01N29/24 |
| A,D | JP 2005 021380 A (TOSHIBA CORP)<br>27 January 2005 (2005-01-27)<br>* abstract; figures 1, 2 *<br>* paragraph [0041] - paragraph [0042] *<br>* paragraph [0077] - paragraph [0082] *<br>* paragraph [0087] - paragraph [0097] *<br>* paragraph [0103] - paragraph [0104] *<br>& EP 1 493 380 A1 (TOSHIBA KK [JP])<br>5 January 2005 (2005-01-05)<br>* abstract; figures 1, 2 *<br>* paragraph [0038] - paragraph [0039] *<br>* paragraph [0071] - paragraph [0075] *<br>* paragraph [0080] - paragraph [0089] *<br>* paragraph [0095] - paragraph [0096] *<br>----- | 1-6 | |
| A | WO 2007/072490 A1 (ULTRAVIEW LTD [IL]; BRUCK ABRAHAM [IL])<br>28 June 2007 (2007-06-28)<br>* abstract; figure 4 *<br>* page 8, line 16 - line 31 *<br>* page 9, line 14 - page 10, line 3 *<br>----- | 1-6 | TECHNICAL FIELDS SEARCHED (IPC)<br><br>G01S<br>A61B<br>G01N<br>G10K |
| A | WO 2007/148239 A2 (KONINKL PHILIPS ELECTRONICS NV [NL]; BURCHER MICHAEL [US]) 27 December 2007 (2007-12-27)<br>* abstract; figures 1, 2 *<br>* page 5, line 27 - page 7, line 10 *<br>* page 10, line 1 - line 24 *<br>----- | 1-6 | |

The present search report has been drawn up for all claims

| Place of search | Date of completion of the search | Examiner |
|---|---|---|
| Munich | 20 June 2017 | Knoll, Bernhard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 15 5970

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2017

| Patent document cited in search report | | | Publication date | Patent family member(s) | | Publication date |
|---|---|---|---|---|---|---|
| WO 2009154298 | A1 | | 23-12-2009 | CN 102065748 A | | 18-05-2011 |
| | | | | CN 103099644 A | | 15-05-2013 |
| | | | | EP 2303103 A1 | | 06-04-2011 |
| | | | | JP 5294998 B2 | | 18-09-2013 |
| | | | | JP 2010022816 A | | 04-02-2010 |
| | | | | US 2011088477 A1 | | 21-04-2011 |
| | | | | US 2015164472 A1 | | 18-06-2015 |
| | | | | WO 2009154298 A1 | | 23-12-2009 |
| JP 2005021380 | A | | 27-01-2005 | CA 2435990 A1 | | 02-01-2005 |
| | | | | CN 1575770 A | | 09-02-2005 |
| | | | | EP 1493380 A1 | | 05-01-2005 |
| | | | | JP 4406226 B2 | | 27-01-2010 |
| | | | | JP 2005021380 A | | 27-01-2005 |
| | | | | KR 20050003948 A | | 12-01-2005 |
| | | | | KR 20060080562 A | | 10-07-2006 |
| | | | | US 2005004458 A1 | | 06-01-2005 |
| WO 2007072490 | A1 | | 28-06-2007 | NONE | | |
| WO 2007148239 | A2 | | 27-12-2007 | BR PI0713454 A2 | | 08-01-2013 |
| | | | | CN 101472520 A | | 01-07-2009 |
| | | | | EP 2034878 A2 | | 18-03-2009 |
| | | | | JP 5432708 B2 | | 05-03-2014 |
| | | | | JP 2009540904 A | | 26-11-2009 |
| | | | | TW 200808272 A | | 16-02-2008 |
| | | | | US 2009187099 A1 | | 23-07-2009 |
| | | | | WO 2007148239 A2 | | 27-12-2007 |

EPO FORM P0459

For more details about this annex : see Official Journal of the European Patent Office, No. 12/82

**EP 3 199 973 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005021380 A
- JP 2010031171 A
- EP 11706365 A
- EP 2536338 A

**Non-patent literature cited in the description**

- **SRIRANG MANOHAR ; SUSANNE E. VAARTJES ; JOHAN C. G. VAN HESPEN ; JOOST M. KLAASE ; FRANK M. VAN DEN ENGH ; WIENDELT STEENBERGEN ; TON G. VAN LEEUWEN.** Initial results of in vivo non-invasive cancer imaging in the human breast using near-infrared photoacoustics. *Opt. Express,* 2007, vol. 15, 12277-12285